(19) Europäisches Patentamt
European Patent Office
Office européen des brevets

(11) **EP 3 016 613 B1**

(12) **EUROPEAN PATENT SPECIFICATION**

(45) Date of publication and mention
of the grant of the patent:
**10.10.2018 Bulletin 2018/41**

(51) Int Cl.:
*A61L 27/20* [(2006.01)]    *A61L 27/54* [(2006.01)]
*A61L 27/56* [(2006.01)]    *A61L 27/60* [(2006.01)]

(21) Application number: **14819993.8**

(22) Date of filing: **03.07.2014**

(86) International application number:
**PCT/US2014/045456**

(87) International publication number:
**WO 2015/003155 (08.01.2015 Gazette 2015/01)**

(54) **BIO-COMPATIBLE APITHERAPEUTIC NANOFIBERS**

BIOKOMPATIBLE APITHERAPEUTISCHE NANOFASERN

NANOFIBRES APITHÉRAPEUTIQUES BIOCOMPATIBLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.07.2013 US 201361842515 P**

(43) Date of publication of application:
**11.05.2016 Bulletin 2016/19**

(73) Proprietor: **The American University in Cairo
New York, NY 10018-2729 (US)**

(72) Inventors:
• **AZZAZY, Hassan, Mohamed, El-Said
Alexandria 21500 (EG)**
• **SARHAN, Wesam, Awad, Ahmed
Zagazig
Sharkia 44519 (EG)**

(74) Representative: **FRKelly
27 Clyde Road
Dublin D04 F838 (IE)**

(56) References cited:
WO-A1-2013/035072    GB-A- 2 484 319
US-A1- 2010 303 891   US-A1- 2010 303 891
US-B2- 7 357 950      US-B2- 7 357 950

• SALALHA W ET AL: "Encapsulation of bacteria
and viruses in electrospun nanofibres",
NANOTECHNOLOGY, IOP, BRISTOL, GB, vol. 17,
no. 18, 28 September 2006 (2006-09-28), pages
4675-4681, XP020104092, ISSN: 0957-4484, DOI:
10.1088/0957-4484/17/18/025

• GENG X ET AL: "Electrospinning of chitosan
dissolved in concentrated acetic acid solution",
BIOMATERIALS, ELSEVIER SCIENCE
PUBLISHERS BV., BARKING, GB, vol. 26, no. 27,
1 September 2005 (2005-09-01), pages 5427-5432,
XP027768194, ISSN: 0142-9612 [retrieved on
2005-09-01]

• WANG TAO ET AL: "Hydrogel sheets of chitosan,
honey and gelatin as burn wound dressings",
CARBOHYDRATE POLYMERS, vol. 88, no. 1, 28
November 2011 (2011-11-28), pages 75-83,
XP028885578, ISSN: 0144-8617, DOI:
10.1016/J.CARBPOL.2011.11.069

• JAVAD JAFARI ET AL: "Electrospun
chitosan-gelatin nanofiberous scaffold:
fabrication and in vitro evaluation",
BIO-MEDICAL MATERIALS AND ENGINEERING,
vol. 21, no. 3, 1 January 2011 (2011-01-01), pages
99-112, XP055324971, NL ISSN: 0959-2989, DOI:
10.3233/BME-2011-0660

• GENG ET AL.: 'Electrospinning of chitosan
dissolved in concentrated acetic acid solution'
BIOMATERIALS vol. 26, no. 27, 2005, pages 5427
- 5432, XP027768194

• WANG ET AL.: 'Hydrogel sheets of chitosan,
honey and gelatin as burn wound dressings'
CARBOHYDRATE POLYMERS vol. 88, no. 1,
2012, pages 75 - 83, XP028885578

• SALALHA ET AL.: 'Encapsulation of bacteria and
viruses in electrospun nanofibres'
NANOTECHNOLOGY vol. 17, no. 18, 2006, pages
4675 - 4681, XP020104092

- **JAFARI ET AL.: 'Electrospun chitosan-gelatin nanofiberous scaffold: Fabrication and in vitro evaluation' BIO-MEDICAL MATERIALS AND ENGINEERING vol. 21, no. 2, 2011, pages 99 - 112, XP055324971**

**Description**

**FIELD OF THE INVENTION**

This invention relates to tissue engineering, cell culture, antimicrobial dressings and packaging. In particular, the invention relates to garlic apitherapeutic nanofibers for drug delivery, bacteriophage delivery and storage, wound dressing, cancer treatment, HIV treatment, cardiovascular treatment, weight loss and antibacterials.

**BACKGROUND OF THE INVENTION**

Nanofibers can be fabricated using different natural and synthetic polymers with dimensions of less than 100 nanometer to 1000 microns. They have the advantage of having high porosity, high surface to volume ratio and ease of loading with multiple materials leaving to various applications for example in the biomedical field. The tissue engineering of the nanofibers in the present invention advances the art towards applications for drug delivery, bacteriophage delivery and storage, wound dressing, cancer treatment, HIV treatment and weight loss.

**SUMMARY OF THE INVENTION**

The present disclosure provides a nanofibrous scaffold as detailed in claim 1. Advantageous features are provided in dependent claims.
In another embodiment, the (i) chitosan, the (ii) oligochitosan or the (iii) combination of chitosan and oligochitosan is in the range of 1.5% to 5% of the total weight of the nanofibrous scaffold.
The nanofibrous scaffold can also be crosslinked. A range of other materials can be added such as:

- Cells, bacteria, antimicrobial peptides, cell wall hydrolases, enzymes, bacteriophages, or any combination thereof.
- Apitherapeutics (e.g. propolis, beevenom, beeswax, or royal jelly).
- Curcumin (turmeric), alovera, olive oil, olive extract or chamomile.
- Vinegar as apple cidar vinegar.
- Biocompatible polymers (e.g. BSA, gelatin, collagen, alginate, PVA, PHB or PLGA).
- Oxacillin, ciprofloxacin or penicillin.
- Compounds capable of generating nitric oxide (e.g. S-nitroso glutathione).
- Anticancer drugs (e.g. paclitaxel and doxorubicin either in powder, solution, spray-dried, lyophilized or nanoformulation).

**BRIEF DESCRIPTION OF THE DRAWINGS**

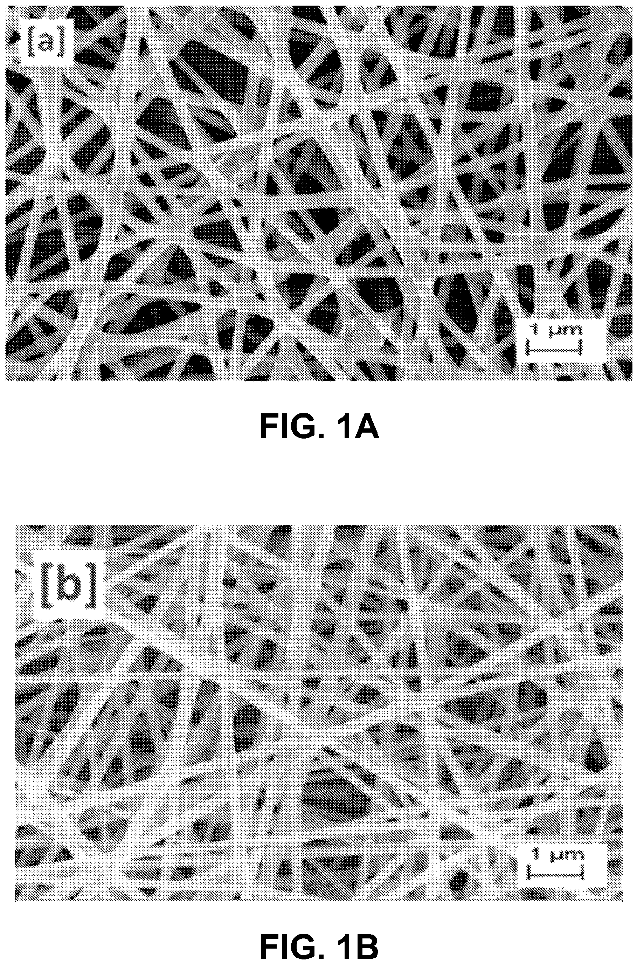

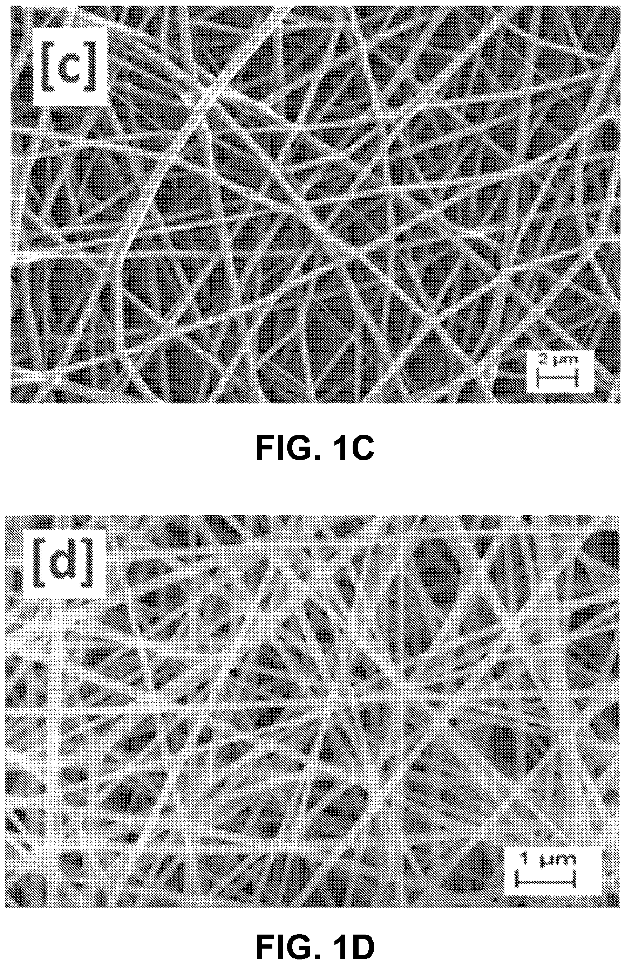

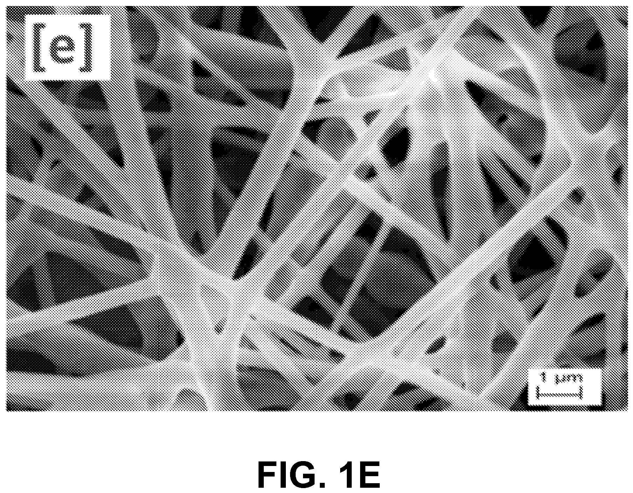

**FIGs. 1A-D** show according to an exemplary embodiment of the invention SEM images of electrospun chitosan (CH), PVA nanofibers with increasing concentrations of honey (NBM1) reaching to 40% (I added the figure of the 40%). CH/PVA/NBM1: **FIG. 1A** 3.5%/7%/15%, **FIG. 1B** 3.5%/7%/20%, **FIG. 1C** 3.5%/7%/27%, and **FIG. 1D** 3.5%/7%/30%, **FIG. 1E** 3.5%/7%/40%. The inclusion of honey affected the viscosity as well as the morphology of the developed nanofibers.

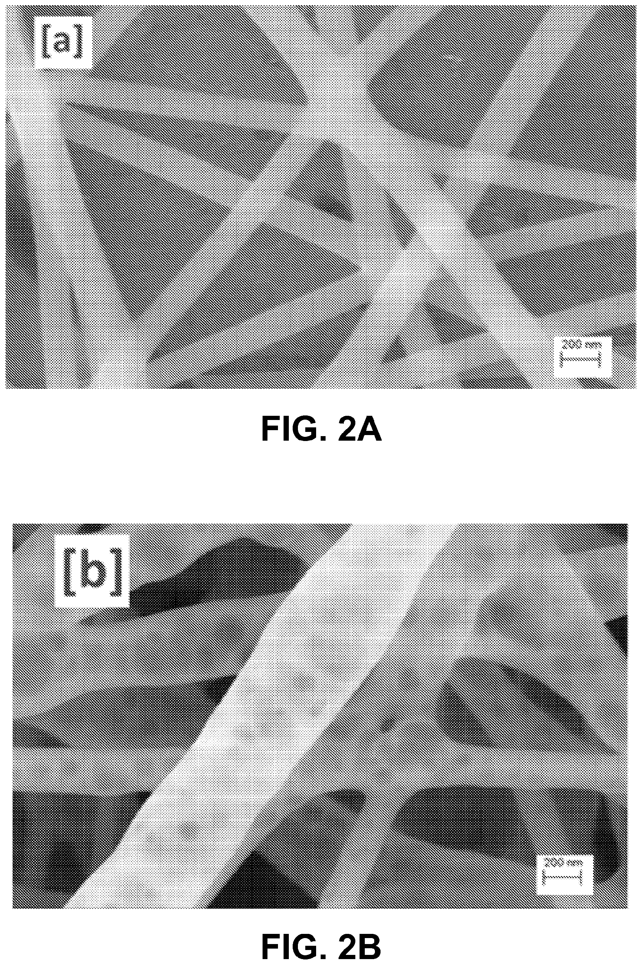

**FIGs. 2A-B** show according to an exemplary embodiment of the invention SEM images showing the effect of inclusion of honey on the morphology of the nanofibers of 1.5% chitosan and PVA (7%). **FIG. 2A** Chitosan (1.5%)/ PVA (7%), **FIG. 2B** Chitosan (1.5%)/ PVA(7%)/ Honey (30%).

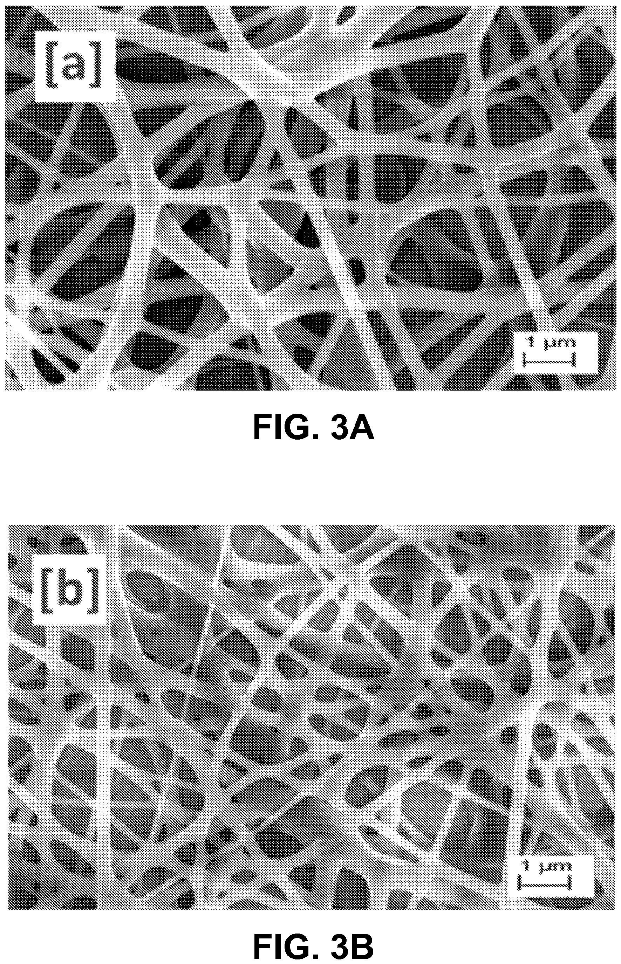

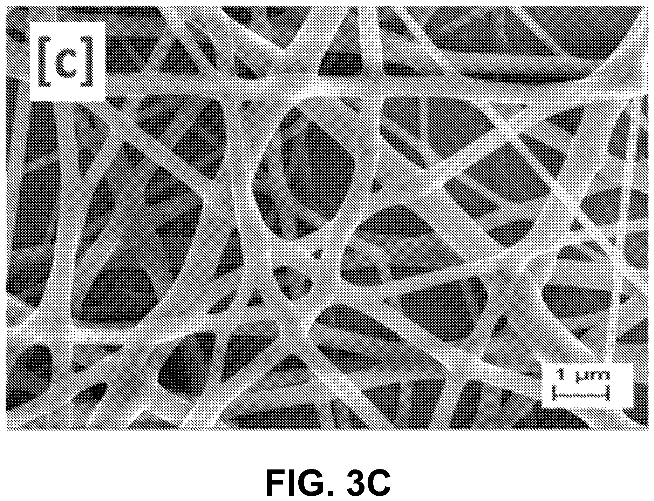

**FIGs. 3A-C** show according to an exemplary embodiment of the invention SEM images of 7% PVA and 30% honey nanofibers with increasing concentrations of chitosan in the presence of (10%) garlic extract. **FIG. 3A** 5% chitosan, **FIG. 3B** 7%chitosan, **FIG. 3C** 9% chitosan.

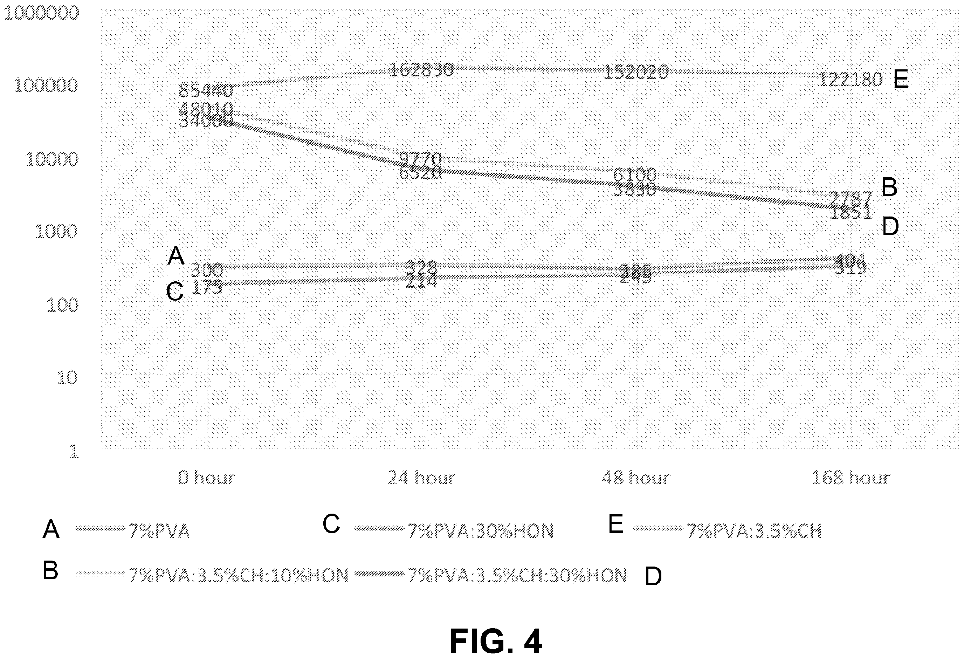

**FIG. 4** shows according to an exemplary embodiment of the invention a decrease in viscosity (mpas) of the PVA/chitosan solutions upon addition of honey with time, i.e. PVA, PVA/HON, PVA/CH and PVA/CH/HON solutions with time. Through the graph it is apparent that addition of honey resulted in a noticeable decrease in viscosity of the solution. This decrease in viscosity of the PVA/CH/HON solutions could be due to the enzymatic degradation of chitosan via the enzymes present in the honey. Small amounts of enzymes occur naturally in honey, the predominant of which are invertase ($\alpha$-glucosidase), diastase (amylase) and glucose oxidase. Such enzymes that transform polysaccharides into smaller products, where, amylases transform starch to other carbohydrates (dextrins, oligo-, di- and monosaccharides). Thus, being a polysaccharide, chitosan is most likely to be affected by such enzymes.

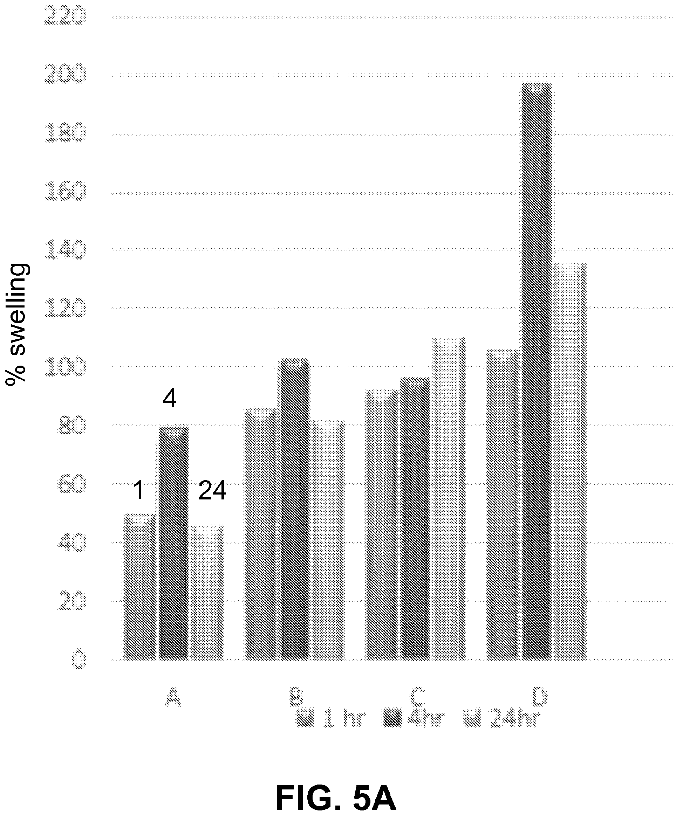

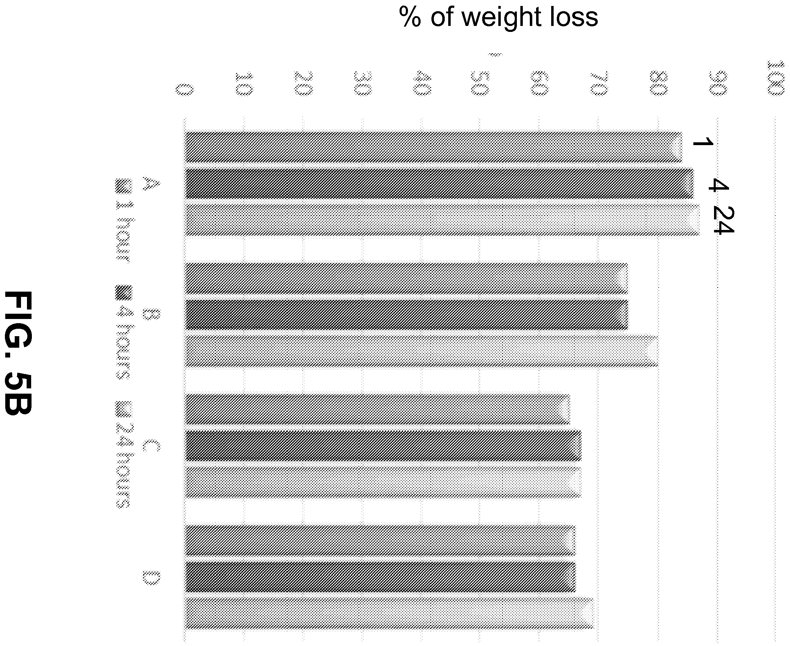

**FIG. 5**    shows according to an exemplary embodiment of the invention the decrease in the viscosity (mpas) of the 7%PVA/3.5%chitosan/30%honey solution upon addition of 10% garlic extract. **FIG. 5** shows that the addition of 10% garlic extract resulted in a massive decrease in the viscosity of the 7%PVA/3,5% chitosan/30%honey from 328 (mpas) to 57 (mpas). Such decrease in viscosity allowed incorporation of greater concentrations of chitosan/oligochitosan reaching to **11%.**using completely biocompatible solvents and avoiding the use of any toxic solvents as seen in figure 3. Such concentrations of chitosan/oligochitosan can be further increased via adjusting the garlic extract concentration with the aging time.

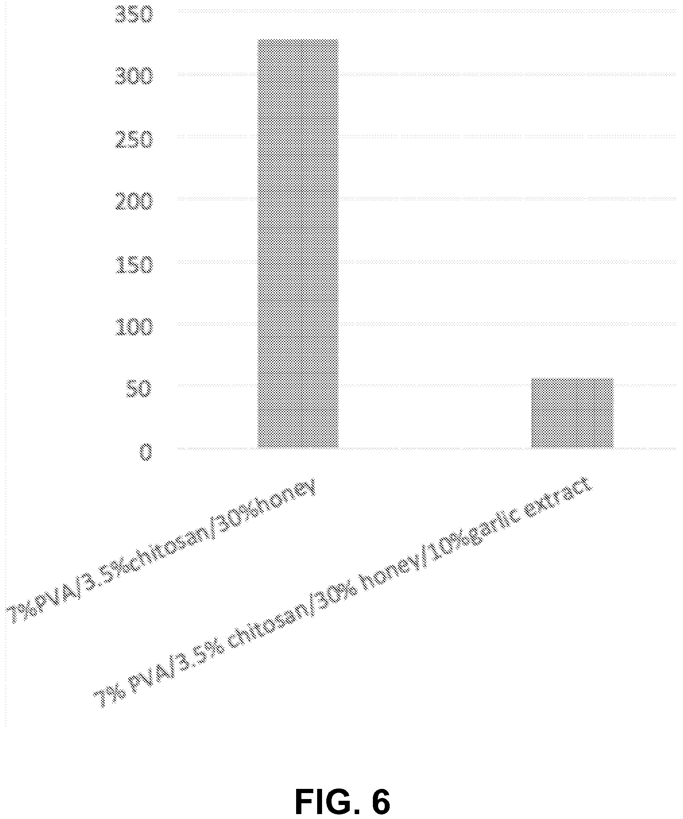

**FIGs. 6 A-B**    show according to an exemplary embodiment of the invention a degree of swelling (%, **FIG. 6A**) and degree of weight loss (%, **FIG. 6B**) of PVA/CH/NBM1 nanofiber mats with different weight ratios of PVA/CH/NBM1 after immersion in PBS (pH 7.4) for 1, 4 and 24 hours. Different weight ratios of PVA/CH/NBM1 tested included: (A) 7%:1.5%:30% (B) 7%:3.5 %:30%, (C) 7%:5.5 %:30%, (D) 7%:3.5 %:20%.

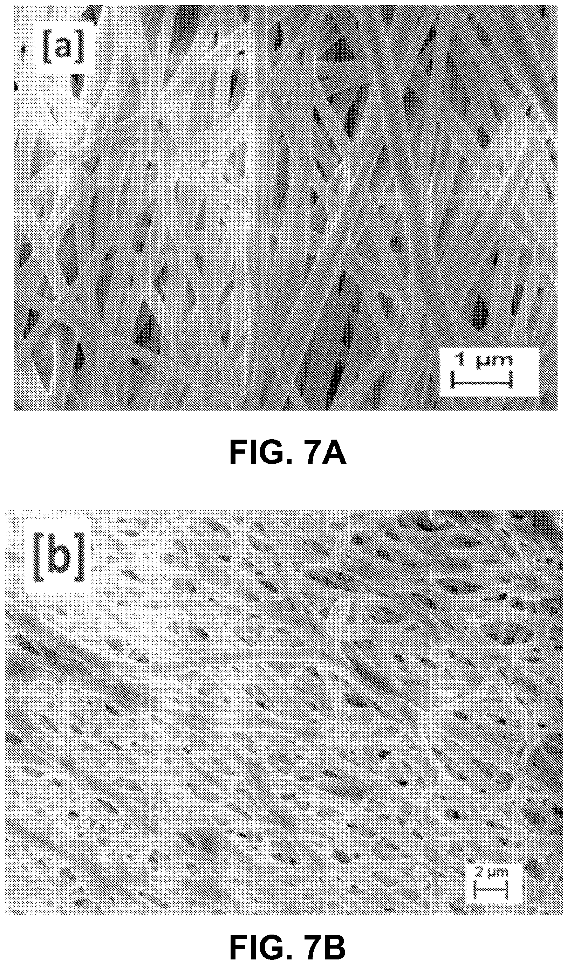

**FIGs. 7A-B**    show according to an exemplary embodiment of the invention SEM images of crosslinked and noncrosslinked PVA, chitosan and honey nanofibers. **FIG. 7A** shows noncrosslinked chitosan/PVA/honey nanofibers. **FIG. 7B** shows crosslinked chitosan/PVA/honey nanofibers.

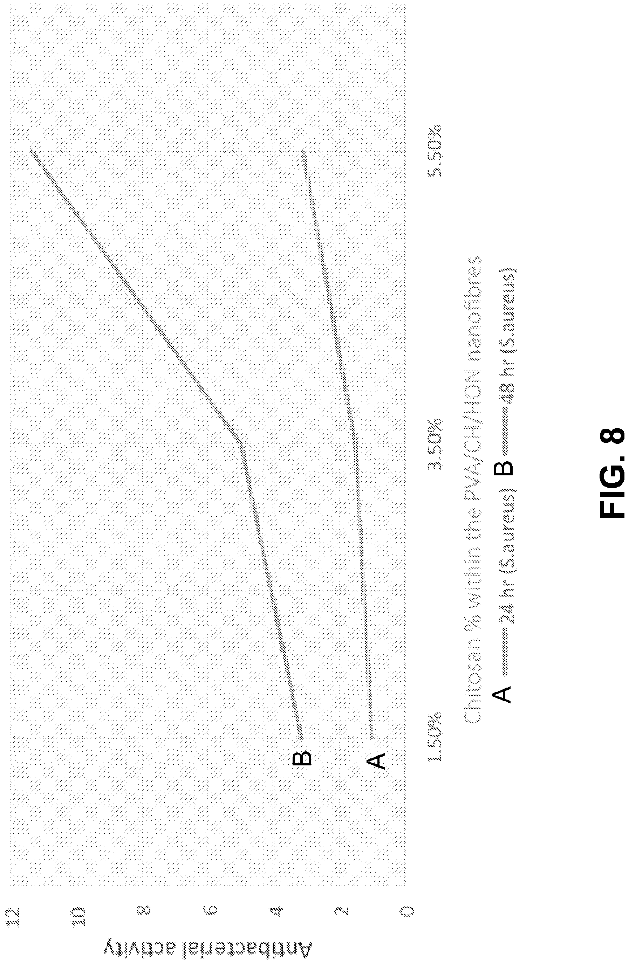

**FIG. 8**    shows according to an exemplary embodiment of the invention the antibacterial activity of the PVA/CH/NBM1 mats against *S.aureus* at 24 and 48 hours on $7 \times 10^8$ CFU/mL bacteria. The weight blending ratios of the e-spun mats were 7% PVA, 30% honey and increasing concentrations of chitosan1.5%, 3.5% and 5.5% chitosan.

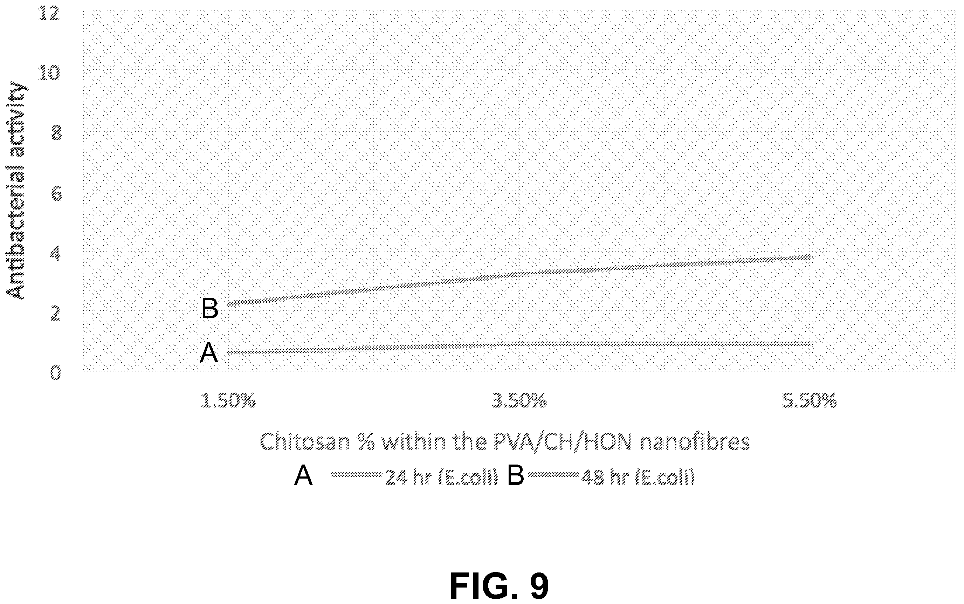

**FIG. 9**    shows according to an exemplary embodiment of the invention the antibacterial activity of the PVA/CH/NBM1 nanofibrous mats with different chitosan concentrations against *E.coli* at 24 and 48 hours on $7 \times 10^8$ CFU/mL bacteria. The weight blending ratios of the e-spun mats were 7% PVA, 30% honey and increasing concentrations of chitosan1.5% 3.5% and 5.5% chitosan.

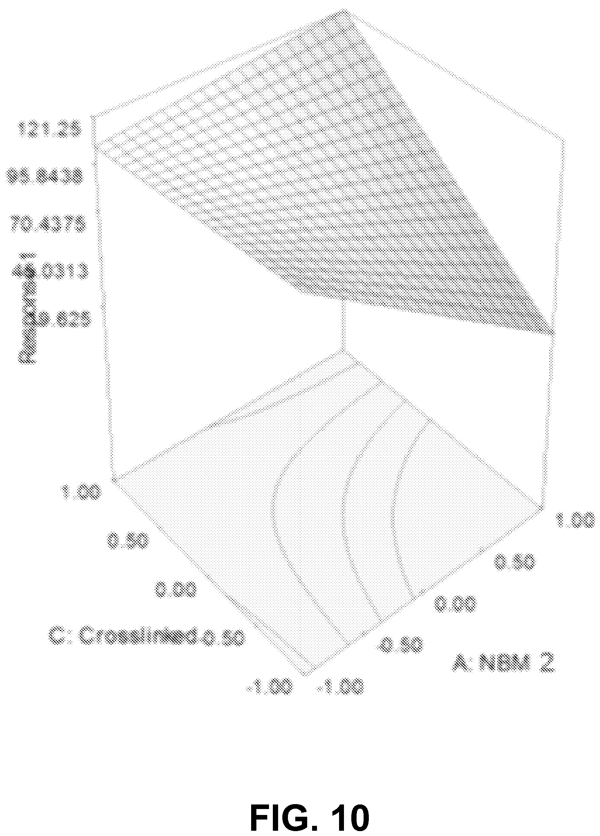

**FIG. 10**    shows according to an exemplary embodiment of the invention a 3D surface plot representing the effect of 10% garlic extract (NBM2), crosslinking and their interaction on swelling of the nanofibrous wound dressing.

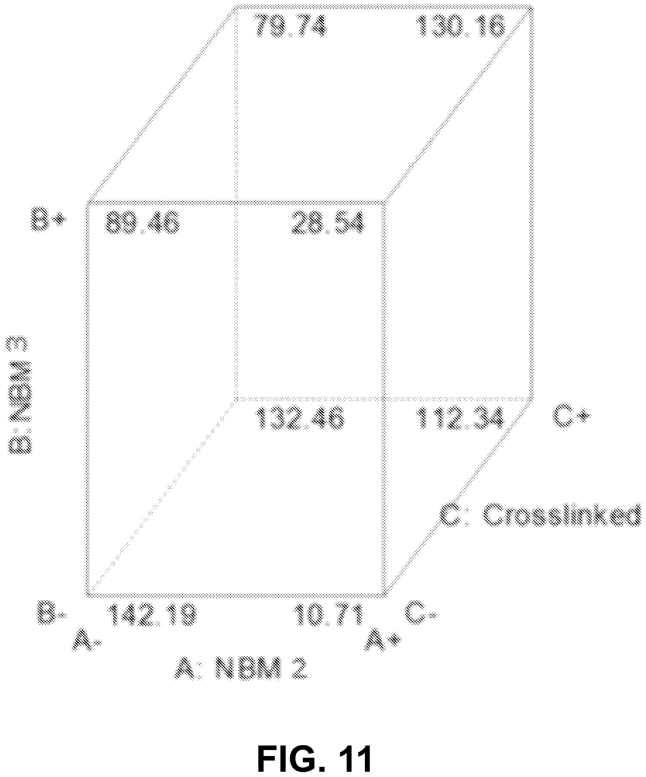

**FIG. 11**    shows according to an exemplary embodiment of the invention a cube plot of the effect of NBM2, NBM3, crosslinking and their interactions on swelling of the nanofibrous wound dressing.

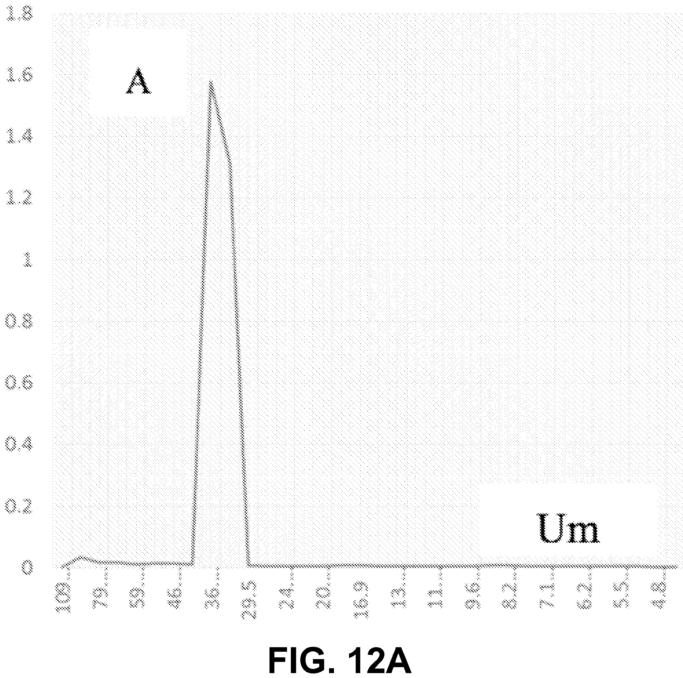

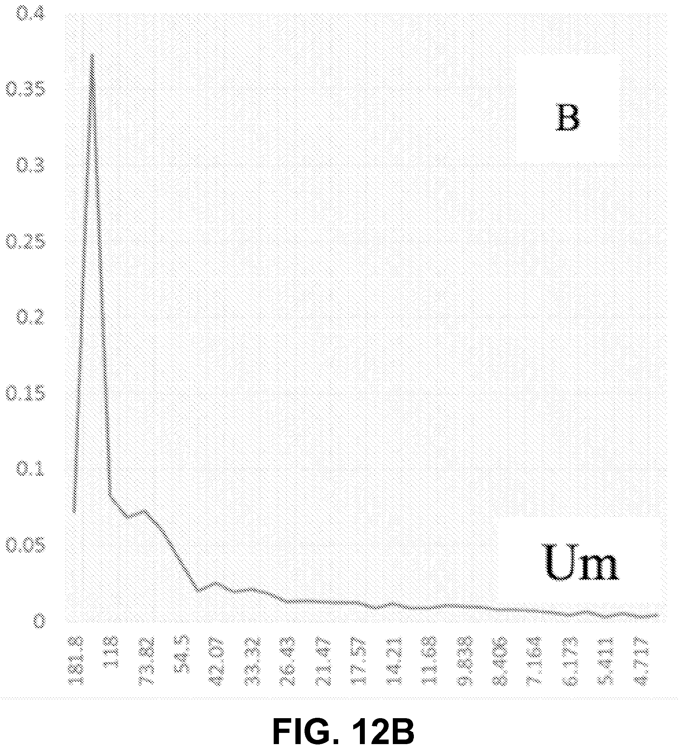

**FIGs. 12A-B**    show according to an exemplary embodiment of the invention pore diameter versus delta volume for the 3.5% chitosan/30%honey (**FIG. 12A**) and pore diameter versus delta volume for the 3.5% chitosan/30% honey and garlic extract (NBM2) (**FIG. 12B**).

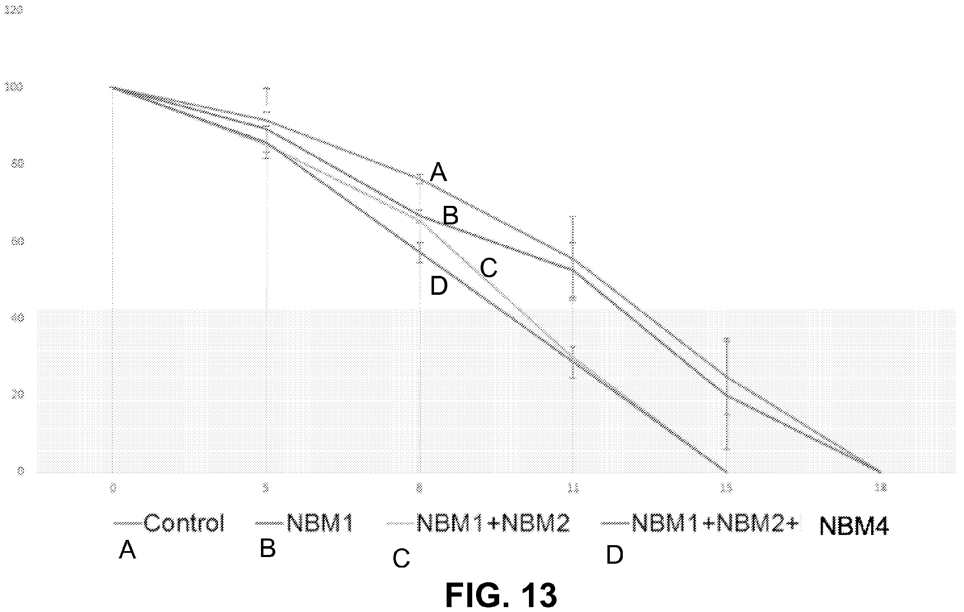

**FIG. 13**    shows according to an exemplary embodiment of the invention the effect of different chitosan nanofiberous nanobiotic wound dressings on the decrease in wound area of a 5mm full excisional wound done on the dorsal back of mice. Measurements were performed at fixed time intervals over a time period of 21 days. The data are presented as mean±S.D. (*N*= 3).

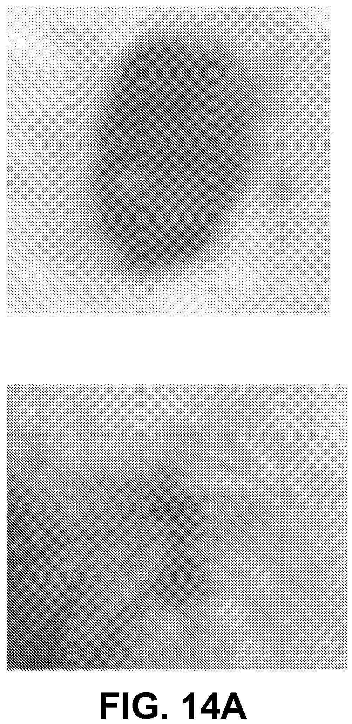

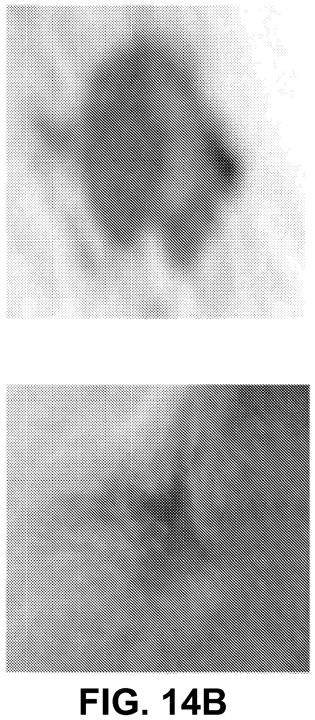

**FIGs. 14A-B**    show according to an exemplary embodiment of the invention images of the wound treated with the nanofibrous dressing composed of PVA/chitosan/honey loaded with garlic extract (NBM2) and vinegar (NBM4) and the control at day zero and day 15. **FIG. 14A** shows PVA/chitosan/honey loaded with (NBM2) and (NBM4). **FIG. 14B** shows a control.

## DETAILED DESCRIPTION

The present invention provides a nanofibrous scaffold (matrix) made of one or more natural and opt. synthetic polymers. The fibers of the scaffold range from less than 100 nanometers to several micrometers and are loaded with natural or synthetic therapeutics. The natural therapeutics are based on garlic and apitherapeutics (including propolis, bee venom, beeswax, royal jelly, their extracts or/and their nanoparticles), and honey reaching to greater than 50% of the total weight of the fibrous mat/scaffold (50% of the nanofibrous mat/scaffold/dressing is honey) and combinations thereof with or without bacteriophages.

The nanofibers can be used for wound dressing, tissue engineering in-vivo and in-vitro, dermal substitutes, antibacterial food packaging, 3-D cell culture, antimicrobial bed dressings, antimicrobial textiles, anti-adhesives, baby diapers, contraceptives, filters, biosensors, catalytic devices and drug, gene, viral, bacterial, bacteriophage delivery and storage systems. The nanofibers can be used as antibacterials, in the treatment of cancer, cardiovascular diseases, HIV and for weight loss.

The garlic apitherapeutic with or without bacteriophage nanofibers are fabricated using the electrospinner. The electrospun nanofibers are either subsequently crosslinked or not.

## Electrospun Nanofibers

Electrospinning is a facile and efficient method for producing nanofibres. The electrospun fibres usually possess high porosity with a small pore size and a very large surface to volume ratio as well as three dimensional structure that mimics the natural extracellular matrix. Moreover, the morphology of the electrospun nanofibres can be controlled by adjusting the different parameters of the electrospinning process. The electrospinning process depends on applying a high voltage to the droplet of the polymer solution. Electrospinning of the polymer into nanofibers begins when the electrostatic forces applied overcome the surface tension of the polymer solution. A charged jet subsequently travels toward the collector and rapid evaporation of the solvent occurs leading to collection of a nanofibrous mat on the collector.

## Apitherapeutics

Apitherpeutics such as propolis, bee venom, beeswax and honey are natural materials with wide range of biological and therapeutic effects such as local anesthetic, anticoagulant, pain soothing, spasmolytic, hepatoprotective, antibacterial, anti-HIV, anti-carcinogenic, antioxidant, anti-inflammatory, anti-anemic, antifungal and anti-parasitic effects. Thus, different bee products have wide range of applications in different areas of medicine.

Among the wide range of medical applications the wound healing ability of honey is the most popular application. Honey shows strong wound healing activity in addition to its acidic nature, which provides the optimal environment for proliferation of fibroblast cells. Propolis is another bee product of special importance. Propolis shows numerous beneficial effects, among them its antibacterial and antifungal activities are of specific importance, as propolis is regarded as a miracle antibiotic. Propolis and Bee venom also show anti-carinogenic effects.

## Garlic

Garlic is among the oldest documented plants for its medicinal and nutritional effects. Garlic possesses a wide range of therapeutic and nutritional effects that include significant cardiovascular effect via reduction of blood pressure and cholesterol. Garlic has also shown anticancer effects, anti-diabetic effects as well as antiseptic, antioxidant and antibacterial effects.

Garlic medicinal effects are usually attributed to its high content of sulfur containing compounds, from which allicin represent 70 to 80%. Garlic can be consumed either dry, fresh, extract or as garlic oil.

## Bacteriophages

Bacteriophages are viruses that infect bacteria and end in their rapid destruction and due to the continuing cycles of co-evolution of phages and bacterial hosts a remarkable diversity of phages coexists. Bacteriophages are specific antibacterials, where each phage can only infect a specific bacterial strain. Bacteriophages were well recognized and used before the discovery of antibiotics, however with the introduction of penicillin phage therapy ceased in the western world but continued actively in some eastern countries. With the alarming rise of bacterial resistance interest in phage therapy was renewed in Western countries.

Bacteriophages are potent antibacterial viruses that can be loaded within the nanofibrous matrix with one or more of the apitherapeutics to increase antibacterial efficiency of the developed nanofibers through synergistic effects.

Nanofibers containing bacteriophages have been successfully spun while maintaining the viability of bacteriophages. However, spinning bacteriophages with other natural or synthetic antibacterials as antibiotics and thus leading to phage antibiotic synergy has not been explored. At the same time delivering phage to the site of infection in such combination nanofibers will result in broader spectrum of antibacterial activity thus overcoming phage specificity, which is one of the major limitations of phage therapy.

The object of the present invention is to produce biocompatible nanofibers based on garlic and apitherapeutics with or without bacteriophages together with other natural materials.

Example 1:

a) Fabrication of nanofibers with increasing concentration of honey (NBM1) reaching to 40% or 50% of the total weight of the scaffold using biocompatible solvent.

b) Fabrication of nanofibers with increasing concentration of chitosan (reaching to 9% or 11% of the total weight of the scaffold) and 30% honey using garlic extract solvent.

Chitosan/oligochitosan and PVA were electrospun with increasing concentrations of honey via biocompatible solvents. Furthermore, increasing concentration of chitosan/oligochitosan and PVA were electrospun with high concentration of honey in the presence of garlic extract.

Prior studies have electrospun chitosan into fibres with diameter of ~100 nm.

However, the main drawback with such fibres was the inability to electrospin chitosan from its aqueous solution due to its high viscosity in addition to the strong hydrogen bonds forming in 3D networks and thus preventing the movement of the polymeric chains of chitosan under the influence of the electrical field. Such a character that forced either the use of toxic solvents to allow spinning of chitosan in considerable high concentrations reaching to 7% upon using (90%) concentrated acetic acid, or reaching 3% upon using trifloroacetic acid and dichloromethane. However, residues of such organic acids and high concentration toxic acids are not favourable especially in applications where totally biocompatible material is required as tissue engineering, drug delivery and wound dressings. To decrease the toxicity of the solvents Charernsriwilaiwat et al. (2010, 2011) prepared chitosan in aqueous salt as CS-hydroxybenzotriazole /PVA and CS-ethylenediaminetetraacetic acid /PVA, however the incorporated chitosan did not exceed 1%.

Another approach for spinning chitosan was through co-spinning with other easily spun polymers in more biocompatible solvents. Among them, the composite PVA/CH have received scientific interest either alone or loaded/mixed with different materials to alter their characters to be suitable for different applications. Still such an approach did not allow spinning of high chitosan concentrations.

Through the present invention the combination of honey, garlic and chitosan shows the capability of controlling the viscosity of the spun solution via the effect of honey and garlic on the molecular weight of chitosan as proofed by the change in viscosity.

Embodiments of the present invention focus on the combination of garlic, honey and chitosan/oligochitosan that allowed reaching the optimum viscosity that is essential for spinning such high concentrations of chitosan/oligochitosan and honey using biocompatible solvents. The combination of honey and chitosan/oligochitosan was the critical factor needed to spin high concentrations of honey. The combination of honey, garlic and chitosan/oligochitosan was the critical factor needed to spin high concentrations of both honey and chitosan/oligochitosan without the use of toxic solvents.

Through the present invention inclusion of garlic extract and honey allowed for decreasing the ultimate high viscosity of chitosan via decreasing its molecular weight to lower molecular weight chitosan/oligochitosan. Thus through the present invention inclusion of these natural materials within a high concentrated highly viscous chitosan solution allowed it to reach upon aging the ultimate viscosity required for electrospinning. Consequently, the combinations presented through the current patent allowed for spinning high concentrations of chitosan reaching to 11% without using any kind of toxic or highly concentrated acidic solvents unlike what was presented in the art where 7% chitosan required 90% acetic acid to be spun as described in the work of Geng et al (2005), and 3% chitosan were spun using trifloroacetic acid which is toxic as described in the work of Ohkawa et al. (2004).

Through the present invention the combinations presented allowed to overcome the low viscosities of honey solutions that made it completely impossible in the prior art to spin honey in more than 3% of the total weight of the fibrous scaffold.

It is noted that in 2012, Maleki et al. produced honey/PVA nanofibers, however the maximum concentration that could be electrospun was 2.25% honey (represented as 30% of 7.5% PVA) of the total weight of the nanofibrous scaffold. Through Malekis' work when the concentration of honey increased to 3% honey, spindle like fibers appeared. According to Malekis' work when the concentration of honey increased in the solution above 3% it decreased the viscosity of the solution making it below the viscosity required for electrospinng. Thus in Malakis work when the concentration of honey increased more than 3% it was impossible for the solution to be spun into fibers. Along the same line, UK patent application (GB 2484319 A) showed that upon spinning 1.5:0.5 honey to polymer uniform fibers could not be produced and consequently they specified in claim no 6 that the maximum inclusion of honey for production of uniform nanofibers is 1: 0.5 honey to polymer.

In the present invention, via the critical combinations described within, nanofibers comprising honey reaching to 50% (3.8: 0.5 honey to polymer) of the total weight of nanofibrous scaffold/mat were fabricated. The critical combinations presented in the present invention allowed fabrication of nanofibrous scaffold having 50% of its total weight honey, where it was impossible in the prior art described through Malakis work to fabricate nanofibrous scaffold having 3% honey.

Fabrication of nanofibers with increasing concentration of honey (NBM1) reaching to 50% of the total weight of the scaffold was achieved via the following procedure: Solutions of chitosan (1.5%, 3.5%, 5,5%) (chitoclear, cg110, TM 3728; premix) and PVA (5% & 7%) (85,000 Mwt; Sigma Aldrich) which are natural and biocompatible synthetic polymers respectively were mixed with increasing concentrations of honey reaching to 40% or 50% using biocompatible solvents containing apple cidar vinegar (10%).

Fabrication of nanofibers with increasing concentration of chitosan/oligochitosan (reaching to 11% of the total weight of the scaffold) and 30% honey via biocompatible solvents was achieved using increasing concentration of garlic extract solvent (from 2% to 30%). Solutions of chitosan (5%, 7% and 9%, 11%) and PVA (7%) were mixed with 30% honey using different concentrations of garlic extract solvents (2% to 30%) containing 10% apple cidar vinegar.

Each of the as-prepared solutions were aged for 2 days at room temperature. The aged solutions were then loaded in a 5 ml plastic syringe attached to a 22 gauge stainless steel needle as the nozzle. The loaded polymer solution was electrospun into nanofibers via the electrospinner (E-spin tech, India) by applying a high voltage (Gamma High Voltage power supply, USA) to charge the polymer solution by attaching a positive polarity electrode to the nozzle and a grounding electrode to the collector. Different voltages were applied to the electrospun polymer solutions to adjust the optimum voltage for each solution. The rate of spinning was maintained at 20 ul/m and the distance between nozzle and collector 20 cm. Samples were collected on a ground and rotary collectors wrapped with aluminum sheet.

Through the present invention, the remarkable combination of chitosan and honey with PVA allowed to reach to the optimum viscosity required for electrospinning, where chitosan increased the decreased viscosity of PVA/HON and at the same time honey decreased the ultimate high viscosities of chitosan/ reaching to optimum improved viscosity with time upon aging. Such an effect that preferentially allowed for incorporating higher chitosan and honey concentrations while using biocompatible solvents. Such concentrations of chitosan that couldn't be reached unless using toxic solvents and haven't yet been reached with honey.

The addition of garlic extract with different concentrations ranging from 2% to 30% resulted in a massive decrease in viscosity.

The reason for the decrease in viscosity produced by garlic and chitosan was investigated. The viscosity average molecular weight of chitosan after addition of different concentrations of garlic (10%, 25%, and 50%) and honey (20%, 30% and 50%) at different time intervals was investigated via factorial design. Viscosity-average molecular weight was determined via viscometric measurements. An Ubbelohde Capillary Viscometer. Measurements were conducted at $25 \pm 0.1°C$. Four consecutive measurements were taken for each sample and the solvent (0.1M Sodium acetate/ 0.5M acetic acid buffer).

The value of the intrinsic viscosity was calculated according to:

$$\text{Relative viscosity, } \eta_{rel} = t/t_s$$

(t is the flow time of the tested solution, ts is the flow time of the solvent)

$$\text{Specific viscosity } \eta_{sp} = (t/t_s)\text{-}1 \quad (4)$$

$$\text{Reduced viscosity } \eta_{red} = \eta_{sp}/c \quad (5)$$

$$\text{Inherent viscosity, } \eta_{inh} = \ln \eta_{red} /C$$

Subsequently the value of the molecular weight of chitosan in each solution was calculated according to the equation $[\eta] = KM\alpha$.

The results (not shown) of the viscometric determination of the molecular weight showed that garlic and honey both degraded chitosan resulting in decrease of its molecular weight, however garlic had the highest impact on the degradation of chitosan decreasing its molecular weight/degrading it to oligochitosan. The results of the factorial experiment further confirmed (ANOVA; $P< 0.05$) the strong positive influence of garlic on the degradation of chitosan.

Inclusion of honey and chitosan with different concentrations affected the properties of the produced nanofibers as. Changing the concentration of chitosan and honey within the nanofibers affected both the swelling and the weight loss properties of the produced nnaofibers.

The nanofibrous matrix could be noncrosslinked or crosslinked allowing for controlled and prolonged release of the loaded materials. Crosslinking prevents the solubility of the developed nanofibers in aqueous solvents according to the degree of crosslinking, thus increasing the potential of the developed nanofibers for different *in-vivo* and *in-vitro* applications.

Crosslinking of the fibrous mats was carried out using both physical and chemical methods of crosslinking. Physical crosslinking was carried out by both heating and freezing/thawing techniques. Physical crosslinking by heating was performed in an oven (Jelotech, OV-11) at both 110 °C, 100 °C for 10 minutes and 80 °C for 25 minutes as well as at 70 °C and 100 °C for 24 hours under vacuum. Whereas physical crosslinking by freezing/ thawing was done by freezing the fiber mats in liquid nitrogen for 15 minutes and thawing for 15 minutes at room temperature for 3 successive cycles. Chemical crosslinking was carried out using glutaraldehyde (GA). The fiber mats were attached to ceramic plate and put in a closed desiccator saturated with the GA vapours (40 ml). The nanofiber mats were exposed to the GA vapours for different time intervals (30, 60, 120 and 180 min). Subsequently, the fibers were heat treated in an oven under vacuum at 70 °C for 24 hours to remove the unreacted GA and enhance the crosslinking reaction.

**Antibacterial effects of the PVA/chitosan/honey nanofibers electrospun via biocompatible solvents.**

Chitosan and honey both show antimicrobial activity. The antibacterial activity of chitosan is based on the interaction of the negatively charged bacterial surfaces with chitosan polycations, consequently the bacterial membrane losses its permeability leading to cell leakage and cell death. Honey on the other hand, exerts its antimicrobial activity via its content of flavanoids, its high sugar content, and acidity in addition to its ability for hydrogen peroxide production. Although, extensive research was done on chitosan nanofibers, no research was done on fibers comprising high concentration of honey and chitosan and testing the effect of their combined antimicrobial activity.

The antibacterial activity of the e-spun PVA/chitosan /NBM1 nanofiberous mats with different concentrations of chitosan [1.5%, 3.5%, and 5.5%] and 7% PVA and 30% honey were tested using *Staphylococcus aureus* and *Escherichia coli* using viable plate count technique. The antibacterial activity was estimated according to the shown formula:

$$\text{Antibacterial activity} = (\log \text{CFU*}\, t - \log \text{CFU*0}) - (\log \text{CFU}t - \log \text{CFU0})$$

where CFU0 and CFUt are the number of colony forming units at time zero and time t for the nanofiberous samples, and

where CFU*0 and CFU*t are the number of colony forming units at time zero and time t for the control.

**The effect of inclusion of higher concentration of garlic extract (NBM2) (10%) and inclusion of propolis (NBM3) and their combinations within the apitherapeutic nanofibers**

Sutjarittangtham et al. and Erdem et al. have co-spun ethanolic extract of propolis spun with polycaprolactone and polyurethane respectively. In the present invention, watersoluble propolis was spun in nanofibers and water soluble propolis was cospun with honey, chitosan/oligochitosan, and PVA (with or without garlic extract, other apitherapeutics and bacteriophages). Furthermore, based on the embodiments of the present invention spinning water soluble propolis rather than ethanolic extract (that was previously spun in the art) ensures more biocompatible nanofibers. In addition, in the present invention propolis was spun within the combination of garlic and biocompatible polymer.

Inclusion of 10% garlic and propolis within the PVA/chitosan and honey nanofibers affected the different properties of the developed nanofibers, as the swelling, weight loss, degradation rate, antibacterial activity, and wound healing ability and cell viability.

Two level factorial design was used to study the effect of inclusion of 10% garlic extract, propolis and crosslinking on the porosity, swelling and cell viability of the apitherapeutic nanofibers of PVA/chitosan and 30% honey.

The two-level factorial design showed that NBM2 and crosslinking had the highest main effects on swelling. NBM2 had negative effect on swelling while crosslinking had positive effect. The interaction of both NBM2 & crosslinking and NBM3 & crosslinking were significant (ANOVA; P< 0.05).

**Inclusion of 10% garlic extract (NBM2) and vinegar (NBM4) within the PVA/chitosan/30% honey nanofibers improves the application of the nanofibers as a wound dressing**

Honey and chitosan are two of the most important materials for wound healing because of their antibacterial properties and also their pronounced effects on wound healing. Chitosan shows stimulating effect on the immune system, analgesic and antimicrobial action and considerable reduction of hyperproliferation of granulation tissue. Whereas honey stimulates the formation of new blood capillaries and stimulate the epithelial cells that form the new skin and produces hydrogen peroxide leading to antibacterial activity also due to its high sugar content.

We conducted an animal study to determine the wound healing ability of the apitherapeutic nanofibers and the effect of the inclusion of 10% garlic extract (NBM2) and vinegar (NBM4) on the wound healing ability was observed. A 5mm full excisional wound was made on the back of 3 mice for each nanofibrous sample and the decrease in wound area was monitored at days 3, 5, 7, 11, 15, 18 and 21. Decrease in wound area was calculated according to the equation $B/A \times 100\%$, where $A$ is the wound area at day zero and $B$ is the wound area at fixed time intervals.

The garlic apitherapeutic nanofiberous scaffold could be used to promote healing and for treatment of chronic wounds as diabetic foot infections, burns and all types of ulcers, as well as for surgical and traumatic wounds.

Upon applying embodiments of the invention as a wound dressing, the nanofiberous matrix made of chitosan and PVA begins degrading and so lead to prolonged release of these components to the wound site and at the same time allow the release of the contained apitheraputics with or without the bacteriophages. The degradation and simultaneous release of the components of the matrix and those contained allows for the antibacterial activities and wound healing properties of them to be exerted on the applied wound for prolonged periods of time. At the same time the nanofiberous form of the dressing resembling the extracellular matrix of the skin promote migration and growth of cells, improve haemostasis, improve adsorption of exudates and facilitate cell respiration due to their porosity. Additionally, such wound dressing allows for the simultaneous application of wound healing dressing and antimicrobials.

**Inclusion of bacteriophages within the PVA/chitosan/honey nanofibers spun via diluted garlic extract remarkably enhanced the decreased antibacterial effect of the PVA/chitosan/30% honey nanofibers against *E.coli***

Bacteriophages are nature's most potent antibacterials especially against resistant bacterial strains. Dai et al, have reported the spinning of nanofibers containing bacteriophages while maintaining the viability of bacteriophages. The present invention differs in the combination that the bacteriophages are presented within. Here, we spun bacteriophages within the combination of biocompatible polymer, one or more apitherapeutics and garlic. Spinning the bacteriophage within such combination can result in broader spectrum of antibacterial activity and enhanced medicinal properties and better storage of bacteriophages. Such scaffolds can be used as drug delivery, bacteriophage delivery and storage, wound dressing and tissue engineering.

*E.coli* bacteriophages were loaded within the garlic apitherapeutic nanofibers. The garlic apitherapeutic nanofibers maintained the stability and viability of the bacteriophages allowing the phage for complete inhibition of *E.coli* after 1 hour of applying the phage loaded garlic apitherapeutic nanofibers. Whereas the phage deficient garlic apitherapeutic nanofibers showed no inhibition of *E.coli* at 1 hour (not shown).

**MTT assay for evaluating the cytotoxicity of the PVA/chitosan/honey nanofibers loaded with 10% garlic extract (NBM2), propolis (NBM3), and vinegar (NBM4) and their combinations.**

PVA/chitosan/honey nanofibers can be loaded with 10% garlic extract (NBM2), propolis (NBM3), and vinegar (NBM4) and their combinations. Each kind of nanofibrous mat was tested for cytotoxicity in the noncrosslinked and crosslinked form. MTT assays of cell viability (not shown) for the different nanofiberous sacffolds/dressings loaded with different NBMs was preformed. The absorbance was normalized with that of negative control which is taken as 100%. Such results confirm the success of the combination that allowed spinning of high concentrations of honey and chitosan without the use of toxic solvents.

The results showed no cytotoxicity thus such embodiments can be effectively applied in applications requiring biocompatible material.

This nanofiberous scaffold can be used in tissue engineering as dermal substitutes by loading them with cells. This nanofiberous scaffold can also be used as in vivo implants for tissue engineering and drug delivery.

The nanofibrous scaffold fabricated through the present invention, contains high concentrations of therapeutic and nutritional material as garlic, honey, chitosan, apple cidar vinegar, and propolis with or without bacteriophages thus increasing the potential of its application. The nanofibrous scaffold presented through the present invention can further include other apithereutics as bee venom, royal jelly and beeswax. The nanofibours scaffold presented in the present invention can be pressed into nanofibrous tablets to be given for different applications as weight loss, antiseptics/antibacterials and nutraceuticals. They can be further loaded with other natural and synthetic embodiments and used for drug delivery in the treatment of different diseases as cancer (bee venom), HIV (bee venom) and cardiovascular diseases (garlic).

The antibacterial nanofibers presented through the present invention were successfully crosslinked, thus extending its application to antimicrobial packaging and antimicrobial dressings and diapers.

The antibacterial biocompatible combination presented in the present invention was developed in the nanofibrous form thus making it suitable to be used in 2d and 3d cell culture systems according to the kind of collector used in the collection of the nanofibers.

Other applications and uses of embodiments of the invention are in:

- Tissue engineering in vitro as skin substitutes.
- Tissue engineering in vivo.
- Drug delivery.
- Weight loss.
- Bacteriophage delivery and storage (stabilization) systems.
- Bacteriophage broad-spectrum antibacterial fibers.
- Gene delivery.
- Viral and bacterial delivery and storage (stabilization) system.
- Smart antimicrobial packaging material.

- 3D cell culture system.
- Antimicrobial bed dressings.
- Antimicrobial surgical dressings
- Antimicrobial textiles i.e: sports clothing, shoes, baby diapers, napkins, etc.
- Contraceptives.
- Filtration systems i.e: water, air, fuel, etc.

**METHOD OF MAKING**

Fabrication of nanofibers with increasing concentration of honey (NBM1) reaching to 50% of the total weight of the scaffold was achieved via the following procedure: Solutions of chitosan (1.5%, 3.5%, 5,5%) (chitoclear, cg110, TM 3728; premix) and PVA (5% & 7%) (85,000 Mwt; Sigma Aldrich) which are natural and biocompatible synthetic polymers respectively were mixed with increasing concentrations of honey reaching to 50% using biocompatible solvents containing apple cidar vinegar (10%).

Fabrication of nanofibers with increasing concentration of chitosan/oligochitosan (reaching to 11% of the total weight of the scaffold) and 30% honey via biocompatible solvents was achieved using increasing concentration of garlic extract solvent (from 2% to 30%). Solutions of chitosan (5%, 7% and 9%, 11%) and PVA (7%) were mixed with 30% honey using different concentrations of garlic extract solvents (2% to 30%) containing 10% apple cidar vinegar.

Each of the as-prepared solutions were aged for 2 days at room temperature. The aged solutions were then loaded in a 5 ml plastic syringe attached to a 22 gauge stainless steel needle as the nozzle. The loaded polymer solution was electrospun into nanofibers via the electrospinner (E-spin tech, India) by applying a high voltage (Gamma High Voltage power supply, USA) to charge the polymer solution by attaching a positive polarity electrode to the nozzle and a grounding electrode to the collector. Different voltages were applied to the electrospun polymer solutions to adjust the optimum voltage for each solution. The rate of spinning was maintained at 20 $\mu$l/m and the distance between nozzle and collector 20 cm. Samples were collected on a ground and rotary collectors wrapped with aluminum sheet.

**References**

Deitzel, J. M., Kleinmeyer, J., Harris, D., & Beck Tan, N. C. (2001). The effect of processing variables on the morphology of electrospun nanofibers and textiles. Polymer, 42(1), 261-272.

Li, D., & Xia, Y. (2004). Electrospinning of nanofibers: reinventing the wheel?. Advanced materials, 16(14), 1151-1170.

Lawson, L. D. (1998). Garlic: a review of its medicinal effects and indicated active compounds. Blood, 179, 62.

Münstedt, K., & Bogdanov, S. (2009). Bee products and their potential use in modern medicine. Journal of ApiProduct and ApiMedical Science, 1(3), 57-63.

Sforcin, J. M., & Bankova, V. (2011). Propolis: is there a potential for the development of new drugs?. Journal of ethnopharmacology, 133(2), 253-260.

Zumla, A., & Lulat, A. (1989). Honey--a remedy rediscovered. Journal of the Royal Society of Medicine, 82(7), 384.

Charernsriwilaiwat, N., Opanasopit, P., Rojanarata, T., Ngawhirunpat, T., & Supaphol, P. (2010). Preparation and characterization of chitosan-hydroxybenzotriazole/polyvinyl alcohol blend nanofibers by the electrospinning technique. Carbohydrate Polymers, 81(3), 675-680.

Charernsriwilaiwat, N., Rojanarata, T., Ngawhirunpat, T., & Opanasopit, P. (2012). Electrospun chitosan/polyvinyl alcohol nanofibre mats for wound healing. International Wound Journal.

Ohkawa, K., Cha, D., Kim, H., Nishida, A., & Yamamoto, H. (2004). Electrospinning of chitosan. Macromolecular Rapid Communications, 25(18), 1600-1605.

Geng, X., Kwon, O. H., & Jang, J. (2005). Electrospinning of chitosan dissolved in concentrated acetic acid solution. Biomaterials, 26(27), 5427-5432.

Zhang, Y., Huang, X., Duan, B., Wu, L., Li, S., & Yuan, X. (2007). Preparation of electrospun chitosan/poly (vinyl alcohol) membranes. Colloid and Polymer Science, 285(8), 855-863.

Paipitak, K., Pornpra, T., Mongkontalang, P., Techitdheer, W., & Pecharapa, W. (2011). Characterization of PVA-chitosan nanofibers prepared by electrospinning. Procedia Engineering, 8, 101-105.

Maleki, H., Gharehaghaji, A. A., & Dijkstra, P. J. (2013). A novel honey-based nanofibrous scaffold for wound dressing application. Journal of Applied Polymer Science, 127(5), 4086-4092.

Sutjarittangtham, K., Sanpa, S., Tunkasiri, T., Rachtanapun, P., Chantawannakul, P., Intatha, U & Eitssayeam, S. (2012). Preparation of Polycaprolactone/Ethanolic Extract Propolis Nanofibers Films. Advanced Materials Research, 506, 226-229.

Erdem, R., Usta, i., Sancak, E., Kocak, D., & Akalin, M. (2013). Effect of propolis extract to morphology of electrospun polyurethane nanofibers. Nano Studies, 7, 21-26.

Salalha, W., Kuhn, J., Dror, Y., & Zussman, E. (2006). Encapsulation of bacteria and viruses in electrospun nanofibres. Nanotechnology, 17(18), 4675.

Dai, M., Senecal, A., & Nugen, S. R. (2014). Electrospun water-soluble polymer nanofibers for the dehydration and storage of sensitive reagents. Nanotechnology, 25(22), 225101.

Gholipour-Kanani, A., Bahrami, S. H., Samadi-Kochaksaraie, A., Ahmadi-Tafti, H., Rabbani, S., Kororian, A., & Erfani, E. (2012). Effect of tissue-engineered chitosan-poly (vinyl alcohol) nanofibrous scaffolds on healing of burn wounds of rat skin. IET nanobiotechnology, 6(4), 129-135.

Wang, T., Zhu, X. K., Xue, X. T., & Wu, D. Y. (2012). Hydrogel sheets of chitosan, honey and gelatin as burn wound dressings. Carbohydrate Polymers,88(1), 75-83.

## Claims

1. A nanofibrous scaffold for applications requiring biocompatible materials, wherein the nanofibrous scaffold comprises nanofibers with electospun therein via biocompatible solvents:

   (a) in the range of 1.5% to 11% of the total weight of the nanofibrous scaffold (i) chitosan, (ii) oligochitosan or (iii) a combination of chitosan and oligochitosan,
   (b) honey in the range of 25% to 50% of the total weight of the nanofibrous scaffold, and
   (c) garlic extract in the range of 2% to 30% of the total weight of the nanofibrous scaffold.

2. The nanofibrous scaffold as set forth in claim 1, wherein the (i) chitosan, the (ii) oligochitosan or the (iii) combination of chitosan and oligochitosan is in the range of 1.5% to 5% of the total weight of the nanofibrous scaffold.

3. The nanofibrous scaffold as set forth in claim 1, further comprising cells, bacteria, antimicrobial peptides, cell wall hydrolases, enzymes, bacteriophages, or any combination thereof.

4. The nanofibrous scaffold as set forth in claim 1, further comprising apitherapeutics.

5. The nanofibrous scaffold as set forth in claim 4, wherein the apitherapeutics are propolis, beevenom, beeswax, or royal jelly.

6. The nanofibrous scaffold as set forth in claim 1, further comprising curcumin (turmeric), alovera oil or extract, olive oil, olive extract or chamomile.

7. The nanofibrous scaffold as set forth in claim 1, further comprising apple cidar vinegar.

8. The nanofibrous scaffold as set forth in claim 1, further comprising BSA, gelatin, collagen, alginate, PVA, PHB, or PLGA.

9. The nanofibrous scaffold as set forth in claim 1, further comprising oxacillin, ciprofloxacin or penicillin.

10. The nanofibrous scaffold as set forth in claim 1, further comprising compounds capable of generating nitric oxide.

11. The nanofibrous scaffold as set forth in claim 1, where the nanofibrous scaffold is crosslinked.

12. The nanofibrous scaffold as set forth in claim 1, further comprising anticancer drugs.

**Patentansprüche**

1. Nanofasergerüst für Anwendungen, die biokompatible Materialien erfordern, wobei das Nanofasergerüst Nanofasern umfasst, die über biokompatible Lösungsmittel darin Folgendes elektrogesponnen aufweisen:

   (a) in dem Bereich von 1,5 % bis 11 % des Gesamtgewichts des Nanofasergerüsts (i) Chitosan, (ii) Oligochitosan oder (iii) eine Kombination aus Chitosan und Oligochitosan,
   (b) Honig in dem Bereich von 25 % bis 50 % des Gesamtgewichts des Nanofasergerüsts und
   (c) Knoblauchextrakt in dem Bereich von 2 % bis 30 % des Gesamtgewichts des Nanofasergerüsts.

2. Nanofasergerüst wie in Anspruch 1 dargelegt, wobei das (i) Chitosan, das (ii) Oligochitosan oder die (iii) Kombination aus Chitosan und Oligochitosan in dem Bereich von 1,5 % bis 5 % des Gesamtgewichts des Nanofasergerüsts liegt.

3. Nanofasergerüst wie in Anspruch 1 dargelegt, das weiter Zellen, Bakterien, antimikrobielle Peptide, Zellwandhydrolasen, Enzyme, Bakteriophagen oder jedwede Kombination davon umfasst.

4. Nanofasergerüst wie in Anspruch 1 dargelegt, das weiter Apitherapeutika umfasst.

5. Nanofasergerüst wie in Anspruch 4 dargelegt, wobei die Apitherapeutika Propolis, Bienengift, Bienenwachs oder Gelée Royale sind.

6. Nanofasergerüst wie in Anspruch 1 dargelegt, das weiter Curcumin (Gelbwurz), Aloe-Vera-Öl oder -Extrakt, Olivenöl, Olivenextrakt oder Kamille umfasst.

7. Nanofasergerüst wie in Anspruch 1 dargelegt, das weiter Apfelessig umfasst.

8. Nanofasergerüst wie in Anspruch 1 dargelegt, das weiter BSA, Gelatine, Kollagen, Alginat, PVA, PHB oder PLGA umfasst.

9. Nanofasergerüst wie in Anspruch 1 dargelegt, das weiter Oxacillin, Ciprofloxacin oder Penicillin umfasst.

10. Nanofasergerüst wie in Anspruch 1 dargelegt, das weiter Verbindungen umfasst, die Stickstoffmonoxid erzeugen können.

11. Nanofasergerüst wie in Anspruch 1 dargelegt, wo das Nanofasergerüst vernetzt ist.

12. Nanofasergerüst wie in Anspruch 1 dargelegt, das weiter Antikrebs-Arzneimittel umfasst.

**Revendications**

1. Echafaudage nanofibreux destiné à des applications nécessitant des matériaux biocompatibles, où l'échafaudage nanofibreux comprend des nanofibres ayant, électrofilés dans celles-ci via des solvants biocompatibles :

   (a) dans la plage allant de 1,5% à 11% du poids total de l'échafaudage nanofibreux, (i) du chitosane, (ii) de l'oligochitosane ou (iii) une combinaison de chitosane et d'oligochitosane,
   (b) du miel, dans la plage allant de 25% à 50% du poids total de l'échafaudage nanofibreux, et
   (c) de l'extrait d'ail, dans la plage allant de 2% à 30% du poids total de l'échafaudage nanofibreux.

**2.** Echafaudage nanofibreux tel qu'exposé selon la revendication 1, dans lequel (i) le chitosane, (ii) l'oligochitosane ou (iii) la combinaison de chitosane et d'oligochitosane se trouvent dans la plage allant de 1,5% à 5% du poids total de l'échafaudage nanofibreux.

**3.** Echafaudage nanofibreux tel qu'exposé selon la revendication 1, comprenant en outre des cellules, des bactéries, des peptides antimicrobiens, des hydrolases de parois cellulaires, des enzymes, des bactériophages, ou une combinaison quelconque de ceux-ci.

**4.** Echafaudage nanofibreux tel qu'exposé selon la revendication 1, comprenant en outre des substances apithérapeutiques.

**5.** Echafaudage nanofibreux tel qu'exposé selon la revendication 4, dans lequel les substances apithérapeutiques sont constituées de propolis, de venin d'abeille, de cire d'abeille ou de gelée royale.

**6.** Echafaudage nanofibreux tel qu'exposé selon la revendication 1, comprenant en outre de la curcumine (curcuma), de l'huile ou un extrait d'aloe vera, de l'huile d'olive, un extrait d'olive ou de la camomille.

**7.** Echafaudage nanofibreux tel qu'exposé selon la revendication 1, comprenant en outre du vinaigre de cidre de pomme.

**8.** Echafaudage nanofibreux tel qu'exposé selon la revendication 1, comprenant en outre de la SAB, de la gélatine, du collagène, un alginate, du PVA, du PHB ou du PLGA.

**9.** Echafaudage nanofibreux tel qu'exposé selon la revendication 1, comprenant en outre de l'oxacilline, de la ciprofloxacine ou de la pénicilline.

**10.** Echafaudage nanofibreux tel qu'exposé selon la revendication 1, comprenant en outre des composés capables de générer de l'oxyde nitrique.

**11.** Echafaudage nanofibreux tel qu'exposé selon la revendication 1, où l'échafaudage nanofibreux est réticulé.

**12.** Echafaudage nanofibreux tel qu'exposé selon la revendication 1, comprenant en outre des médicaments anticancéreux.

**FIG. 1A**

**FIG. 1B**

**FIG. 1C**

**FIG. 1D**

**FIG. 1E**

**FIG. 2A**

**FIG. 2B**

**FIG. 3A**

**FIG. 3B**

**FIG. 3C**

**FIG. 4**

EP 3 016 613 B1

**FIG. 5A**

FIG. 5B

**FIG. 6**

**FIG. 7A**

**FIG. 7B**

**FIG. 8**

EP 3 016 613 B1

Antibacterial activity

12 10 8 6 4 2 0

B

A

1.50%    3.50%    5.50%

Chitosan % within the PVA/CH/HON nanofibres

A ——— 24 hr (E.coli)    B ——— 48 hr (E.coli)

**FIG. 9**

**FIG. 10**

**FIG. 11**

FIG. 12A

**FIG. 12B**

EP 3 016 613 B1

**FIG. 13**

FIG. 14A

**FIG. 14B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2484319 A

**Non-patent literature cited in the description**

- **DEITZEL, J. M. ; KLEINMEYER, J. ; HARRIS, D. ; BECK TAN, N. C.** The effect of processing variables on the morphology of electrospun nanofibers and textiles. *Polymer,* 2001, vol. 42 (1), 261-272
- **LI, D. ; XIA, Y.** Electrospinning of nanofibers: reinventing the wheel?. *Advanced materials,* 2004, vol. 16 (14), 1151-1170
- **LAWSON, L. D.** Garlic: a review of its medicinal effects and indicated active compounds. *Blood,* 1998, vol. 179, 62
- **MÜNSTEDT, K. ; BOGDANOV, S.** Bee products and their potential use in modern medicine. *Journal of ApiProduct and ApiMedical Science,* 2009, vol. 1 (3), 57-63
- **SFORCIN, J. M. ; BANKOVA, V.** Propolis: is there a potential for the development of new drugs?. *Journal of ethnopharmacology,* 2011, vol. 133 (2
- **ZUMLA, A. ; LULAT, A.** Honey--a remedy rediscovered. *Journal of the Royal Society of Medicine,* 1989, vol. 82 (7), 384
- **CHARERNSRIWILAIWAT, N. ; OPANASOPIT, P. ; ROJANARATA, T. ; NGAWHIRUNPAT, T. ; SUPAPHOL, P. ; 2010.** Preparation and characterization of chitosan-hydroxybenzotriazole/polyvinyl alcohol blend nanofibers by the electrospinning technique. *Carbohydrate Polymers,* vol. 81 (3), 675-680
- **CHARERNSRIWILAIWAT, N. ; ROJANARATA, T. ; NGAWHIRUNPAT, T. ; OPANASOPIT, P.** Electrospun chitosan/polyvinyl alcohol nanofibre mats for wound healing. *International Wound Journal,* 2012
- **OHKAWA, K. ; CHA, D. ; KIM, H. ; NISHIDA, A. ; YAMAMOTO, H.** Electrospinning of chitosan. *Macromolecular Rapid Communications,* 2004, vol. 25 (18), 1600-1605
- **GENG, X. ; KWON, O. H. ; JANG, J.** Electrospinning of chitosan dissolved in concentrated acetic acid solution. *Biomaterials,* 2005, vol. 26 (27), 5427-5432

- **ZHANG, Y. ; HUANG, X. ; DUAN, B. ; WU, L. ; LI, S. ; YUAN, X.** Preparation of electrospun chitosan/poly (vinyl alcohol) membranes. *Colloid and Polymer Science,* 2007, vol. 285 (8), 855-863
- **PAIPITAK, K. ; PORNPRA, T. ; MONGKONTALANG, P. ; TECHITDHEER, W. ; PECHARAPA, W.** Characterization of PVA-chitosan nanofibers prepared by electrospinning. *Procedia Engineering,* 2011, vol. 8, 101-105
- **MALEKI, H. ; GHAREHAGHAJI, A. A. ; DIJKSTRA, P. J.** A novel honey-based nanofibrous scaffold for wound dressing application. *Journal of Applied Polymer Science,* 2013, vol. 127 (5), 4086-4092
- **SUTJARITTANGTHAM, K. ; SANPA, S. ; TUNKASIRI, T. ; RACHTANAPUN, P. ; CHANTAWANNAKUL, P. ; INTATHA, U ; EITSSAYEAM, S.** Preparation of Polycaprolactone/Ethanolic Extract Propolis Nanofibers Films. *Advanced Materials Research,* 2012, vol. 506, 226-229
- **ERDEM, R. ; USTA, I. ; SANCAK, E. ; KOCAK, D. ; AKALIN, M.** Effect of propolis extract to morphology of electrospun polyurethane nanofibers. *Nano Studies,* 2013, vol. 7, 21-26
- **SALALHA, W. ; KUHN, J. ; DROR, Y. ; ZUSSMAN, E.** Encapsulation of bacteria and viruses in electrospun nanofibres. *Nanotechnology,* 2006, vol. 17 (18), 4675
- **DAI, M. ; SENECAL, A. ; NUGEN, S. R.** Electrospun water-soluble polymer nanofibers for the dehydration and storage of sensitive reagents. *Nanotechnology,* 2014, vol. 25 (22), 225101
- **GHOLIPOUR-KANANI, A. ; BAHRAMI, S. H. ; SAMADI-KOCHAKSARAIE, A. ; AHMADI-TAFTI, H. ; RABBANI, S. ; KORORIAN, A. ; ERFANI, E.** Effect of tissue-engineered chitosan-poly (vinyl alcohol) nanofibrous scaffolds on healing of burn wounds of rat skin. *IET nanobiotechnology,* 2012, vol. 6 (4), 129-135
- **WANG, T. ; ZHU, X. K. ; XUE, X. T. ; WU, D. Y.** Hydrogel sheets of chitosan, honey and gelatin as burn wound dressings. *Carbohydrate Polymers,* 2012, vol. 88 (1), 75-83